# EUROPEAN PATENT APPLICATION

(11) **EP 4 591 863 A1**
(43) Date of publication of application: **30.07.2025**
(21) Application number: 23876754.5
(22) Date of filing: 12.10.2023
(51) Int. Cl.: A61K 31/505, A61P 11/00

(54) **METHOD FOR TREATING PNEUMOCONIOSIS**

(30) Priority: 13.10.2022 CN 202211256476
(71) Applicant: Beijing Tide Pharmaceutical Co., Ltd., Beijing 100176 (CN)
(72) Inventor: WANG, Hongjun, Beijing 100176 (CN); ZHONG, Weiting, Beijing 100176 (CN); LI, Jing, Beijing 100176 (CN); LIU, Weina, Beijing 100176 (CN); ZHAO, Jing, Beijing 100176 (CN); ZHAO, Yanping, Beijing 100176 (CN)
(74) Representative: Turner, Craig Robert
(86) International application number: PCT/CN2023/124137
(87) International publication number: WO 2024/078553

(57) **Abstract**

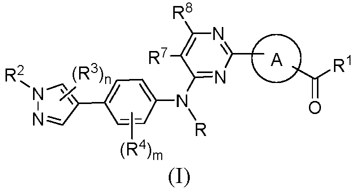

Provided is a method for preventing, relieving, alleviating and/or treating pneumoconiosis, the method comprising administering to an individual in need thereof an effective amount of the compound of formula (I) or a pharmaceutically acceptable salt, ester, stereoisomer, polymorph, solvate, N-oxide, isotope-labeled compound, metabolite or prodrug thereof.

## Description

### FIELD OF THE INVENTION

The present disclosure falls within the field of biological medicine, and specifically relates to a method for the prophylaxis, alleviation, mitigation, and/or treatment of pneumoconiosis, comprising administering to a subject in need thereof an effective amount of a compound of the present application or a pharmaceutically acceptable salt, ester, stereoisomer, polymorph, solvate, N-oxide, isotopically labeled compound, metabolite or prodrug thereof.

### BACKGROUND OF THE INVENTION

Pneumoconiosis is a systemic disease caused by long-term inhalation of industrial dust (particulate matter) during occupational activities and its retention in the lungs. Generally, early-stage pneumoconiosis patients exhibit no significant symptoms, clinical signs, or notable changes in pulmonary function. As the disease progresses, respiratory symptoms gradually manifest, predominantly characterized by chest pain and dyspnea, which may be accompanied by varying degrees of coughing, expectoration, and wheezing.

Due to chronic inhalation of mineral dust, pneumoconiosis patients suffer severe impairment of the respiratory system's clearance and defense mechanisms. Coupled with the chronic and progressive nature of the disease, patients experience markedly reduced resistance, frequently developing complications/comorbidities such as respiratory infections, pneumothorax, pulmonary tuberculosis, chronic obstructive pulmonary disease, bronchiectasis, bronchial asthma, and chronic cor pulmonale, *etc.*

As a severe occupational disease, pneumoconiosis not only inflicts significant physical and psychological harm on patients but also exacerbates social burdens and undermines the stable development of the socioeconomy. To date, no medications or interventions have been definitively and effectively proven to delay or halt the progression of pneumoconiosis.

### SUMMARY OF THE INVENTION

In one aspect, the present disclosure provides a method for the prophylaxis, alleviation, mitigation, and/or treatment of pneumoconiosis, comprising administering to a subject in need thereof an effective amount of a compound of Formula (I) or a pharmaceutically acceptable salt, ester, stereoisomer, polymorph, solvate, N-oxide, isotopically labeled compound, metabolite or prodrug thereof: wherein: 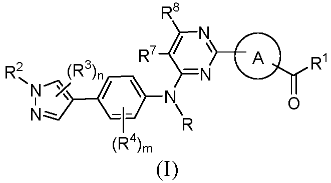
ring A is 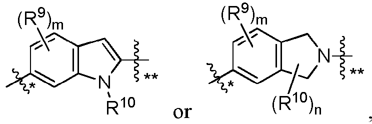 the above group is attached to the pyrimidine ring at either of the two positions labeled * or **, and is attached to the carbonyl group at the other position;
R is selected from the group consisting of H and C₁₋₆ alkyl;
R¹ is 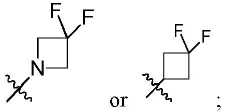
R² is selected from the group consisting of H and C₁₋₆ alkyl;
R³, R⁴, R⁷ and R⁸, at each occurrence, are each independently selected from the group consisting of H, halogen, -NR⁵R⁶, -OH, C₁₋₆ alkyl and -OR⁵;
R⁹ and R¹⁰, at each occurrence, are each independently selected from the group consisting of H, halogen, C₁₋₆ alkyl, C₂₋₆ alkenyl, C₃₋₁₀ cyclic hydrocarbyl, 3-10-membered heterocyclyl, C₆₋₁₀ aryl, 5-14 -membered heteroaryl, C₆₋₁₂ aralkyl, -C(=O)R⁵ and -C₁₋₆ alkylene-O(P=O)(OH)₂;
the above alkylene, alkyl, alkenyl, cyclic hydrocarbyl, heterocyclyl, aryl, heteroaryl and aralkyl, at each occurrence, are each optionally substituted with one or more substituents independently selected from the group consisting of halogen, C₁₋₆ alkyl and -OR⁵;
R⁵ and R⁶, at each occurrence, are each independently selected from the group consisting of H, C₁₋₆ alkyl, C₃₋₁₀ cyclic hydrocarbyl, 3-10-membered heterocyclyl, C₆₋₁₀ aryl, 5-14-membered heteroaryl and C₆₋₁₂ aralkyl;
m, at each occurrence, is each independently an integer of 0, 1, 2 or 3; and
n, at each occurrence, is each independently an integer of 0, 1 or 2.

In another aspect, the present disclosure provides use of the compound of Formula (I) or a pharmaceutically acceptable salt, ester, stereoisomer, polymorph, solvate, N-oxide, isotopically labeled compound, metabolite or prodrug thereof in the manufacture of a medicament for the prophylaxis, alleviation, mitigation, and/or treatment of pneumoconiosis.

In yet another aspect, the present disclosure provides the compound of Formula (I) or a pharmaceutically acceptable salt, ester, stereoisomer, polymorph, solvate, N-oxide, isotopically labeled compound, metabolite or prodrug thereof for use in the prophylaxis, alleviation, mitigation, and/or treatment of pneumoconiosis.

### BRIEF DESCRIPTION OF THE DRAWINGS

Figure 1 shows the pulmonary function index data of silicosis model mice (Note: Compared with the model group, *p < 0.05, **p < 0.01, ****p < 0.0001).
Figure 2 shows the expression levels of TGF-β in the BALF of silicosis model mice in each group (Note: Compared with the model group, ***p < 0.001, ****p < 0.0001).
Figure 3 shows the Masson's Trichrome staining images of lung tissue in silicosis model mice in each group.
Figure 4 shows the silicotic nodule scores in the lungs of silicosis model mice in each group (Note: Compared with the model group, **p < 0.01, ***p < 0.001).
Figure 5 shows the H&E staining images of lung tissue in silicosis model mice in each group.
Figure 6 shows the inflammation scores of lung tissue in silicosis model mice in each group (Note: Compared with the model group, *p < 0.05, **p < 0.01, ****p < 0.0001).

### DETAILED DESCRIPTION OF THE INVENTION

### Definition

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that the following terms will be readily understood by a person skilled in the art, the following definitions are nevertheless put forth to better illustrate the present invention.

The terms "contain", "include", "comprise", "have", or "relate to", as well as other variations used herein are inclusive or open-ended, and do not exclude additional, unrecited elements or method steps.

As used herein, the term "alkylene" refers to a saturated divalent hydrocarbyl, preferably refers to a saturated divalent hydrocarbyl having 1, 2, 3, 4, 5 or 6 carbon atoms, e.g., methylene, ethylene, propylene or butylene.

As used herein, the term "alkyl" is defined as a linear or branched saturated aliphatic hydrocarbon. In some embodiments, alkyl has 1-12, e.g., 1-6, carbon atoms. For example, as used herein, the term "C₁₋₆ alkyl" refers to a linear or branched group having 1-6 carbon atoms (such as methyl, ethyl, n-propyl, isopropyl, n-butyl, isobutyl, sec-butyl, *tert*-butyl*,* n-pentyl, isopentyl, neopentyl, or n-hexyl), which is optionally substituted with one or more (e.g., 1 to 3) suitable substituents such as halogen (in which case the group may be referred to as "haloalkyl") (e.g., CH₂F, CHF₂, CF₃, CCl₃, C₂F₅, C₂Cl₅, CH₂CF₃, CH₂Cl or -CH₂CH₂CF₃ *etc.).* The term "C₁₋₄ alkyl" refers to a linear or branched aliphatic hydrocarbon chain having 1-4 carbon atoms (i.e., methyl, ethyl, n-propyl, isopropyl, n-butyl, isobutyl, sec-butyl or *tert*-butyl)*.*

As used herein, the term "alkenyl" refers to a linear or branched monovalent hydrocarbyl having a double bond and 2-6 carbon atoms ("C₂₋₆ alkenyl"). The alkenyl is e.g., vinyl, 1-propenyl, 2-propenyl, 2-butenyl, 3-butenyl, 2-pentenyl, 3-pentenyl, 4-pentenyl, 2-hexenyl, 3-hexenyl, 4-hexenyl, 5-hexenyl, 2-methyl-2-propenyl and 4-methyl-3-pentenyl. When the compound of the present invention contains an alkenylene group, the compound may exist as the pure E (entgegen) form, the pure Z (zusammen) form, or any mixture thereof.

As used herein, the term "alkynyl" refers to a monovalent hydrocarbyl containing one or more triple bond, and preferably having 2, 3, 4, 5 or 6 carbon atoms, e.g., ethynyl or propynyl.

As used herein, the term "cycloalkyl" refers to a saturated monocyclic or polycyclic (e.g., bicyclic) hydrocarbon ring (e.g., monocyclic, such as cyclopropyl, cyclobutyl, cyclopentyl, cyclohexyl, cycloheptyl, cyclooctyl, or cyclononyl, or bicyclic, including spiro, fused or bridged cyclic system (such as bicyclo[1.1.1]pentyl, bicyclo[2.2.1]heptyl, bicyclo[3.2.1]octyl or bicyclo[5.2.0]nonyl, or decahydronaphthalene *etc.*))*,* which is optionally substituted with one or more (e.g., 1 to 3) suitable substituents. The cycloalkyl has 3 to 15 carbon atoms. For example, the term "C₃₋₆ cycloalkyl" refers to a saturated monocyclic or polycyclic (e.g., bicyclic) hydrocarbon ring having 3 to 6 ring forming carbon atoms (e.g., cyclopropyl, cyclobutyl, cyclopentyl, or cyclohexyl), which is optionally substituted with one or more (e.g., 1 to 3) suitable substituents, e.g., methyl substituted cyclopropyl.

As used herein, the terms "cyclic hydrocarbylene", "cyclic hydrocarbyl" and "hydrocarbon ring" refer to a saturated (i.e., "cycloalkylene" and "cycloalkyl") or unsaturated (i.e., having one or more double and/or triple bonds in the ring) monocyclic or polycyclic hydrocarbon ring having e.g., 3-10 (suitably having 3-8, and more suitably having 3-6) ring carbon atoms, including but not limited to cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), and the like.

As used herein, the terms "heterocyclyl", "heterocyclylene" and "heterocycle" refer to a saturated (i.e., heterocycloalkyl) or partially unsaturated (i.e., having one or more double and/or triple bonds in the ring) cyclic group having e.g., 3-10 (suitably having 3-8, and more suitably having 3-6) ring atoms, wherein at least one ring atom is a heteroatom selected from the group consisting of N, O and S, and the remaining ring atoms are C. For example, "3- to 10-membered heterocyclyl(ene)" of "3- to 10-membered heterocycle" refers to saturated or partially unsaturated heterocyclyl(ene) or heterocycle having 2-9 (e.g., 2, 3, 4, 5, 6, 7, 8 or 9) ring carbon atoms and one or more (e.g., 1, 2, 3, or 4) heteroatoms independently selected from the group consisting of N, O and S. Examples of heterocyclylene, heterocyclyl and heterocycle include, but are not limited to oxiranyl(ene), aziridinyl(ene), azetidinyl(ene), oxetanyl(ene), tetrahydrofuranyl(ene), dioxolinyl(ene), pyrrolidinyl(ene), pyrrolidonyl(ene), imidazolidinyl(ene), pyrazolidinyl(ene), pyrrolinyl(ene), tetrahydropyranyl(ene), piperidinyl(ene), morpholinyl(ene), dithianyl(ene), thiomorpholinyl(ene), piperazinyl(ene) or trithianyl(ene). Said group also encompasses a bicyclic system, including a spiro, fused, or bridged system (e.g., 8-azaspiro[4.5]decane, 3,9-diazaspiro[5.5]undecane, 2-azabicyclo[2.2.2]octane, *etc.*)*.* Heterocyclylene, heterocyclyl and heterocycle may optionally be substituted with one or more (e.g., 1, 2, 3 or 4) suitable substituents.

As used herein, the terms "aryl(ene)" and "aromatic ring" refer to an all-carbon monocyclic or fused-ring polycyclic aromatic group having a conjugated π electron system. For example, as used herein, the terms "C₆₋₁₀ aryl(ene)" and "C₆₋₁₀ aromatic ring" refer to an aromatic group containing 6 to 10 carbon atoms, such as phenyl(ene) (benzene ring) or naphthyl(ene) (naphthalene ring). Aryl(ene) or aromatic ring is optionally substituted with one or more (such as 1 to 3) suitable substituents (e.g., halogen, -OH, -CN, -NO₂, and C₁₋₆ alkyl, *etc.*)*.*

As used herein, the terms "heteroaryl(ene)" and "heteroaromatic ring" refer to a monocyclic, bicyclic or tricyclic aromatic ring system having 5, 6, 8, 9, 10, 11, 12, 13 or 14 ring atoms, particularly 1 or 2 or 3 or 4 or 5 or 6 or 9 or 10 carbon atoms, and containing at least one heteroatom (such as O, N, or S), which can be same to different. Moreover, in each case, it can be benzo-fused. In particular, "heteroaryl(ene)" or "heteroaromatic ring" is selected from the group consisting of thienyl(ene), furyl(ene), pyrrolyl(ene), oxazolyl(ene), thiazolyl(ene), imidazolyl(ene), pyrazolyl(ene), isoxazolyl(ene), isothiazolyl(ene), oxadiazolyl(ene), triazolyl(ene), thiadiazolyl(ene) *etc.,* and benzo derivatives thereof; or pyridinyl(ene), pyridazinyl(ene), pyrimidinyl(ene), pyrazinyl(ene), triazinyl(ene), etc., and benzo derivatives thereof.

As used herein, the term "aralkyl" preferably means aryl or heteroaryl substituted alkyl, wherein aryl, heteroaryl and alkyl are as defined herein. Normally, the aryl group may have 6-14 carbon atoms, the heteroaryl group may have 5-14 ring atoms, and the alkyl group may have 1-6 carbon atoms. Exemplary aralkyl group includes, but is not limited to, benzyl, phenylethyl, phenylpropyl, phenylbutyl.

As used herein, the term "halo" or "halogen" are defined to include F, Cl, Br, or I.

As used herein, the term "nitrogen containing heterocycle" refers to a saturated or unsaturated monocyclic or bicyclic group having 2, 3, 4, 5, 6, 7, 8, 9, 10, 11, 12 or 13 carbon atoms and at least one nitrogen atom in the ring, which may further optionally comprise one or more (e.g., one, two, three or four) ring members selected from the group consisting of N, O, C=O, S, S=O and S(=O)₂. The nitrogen containing heterocycle is attached to the rest of the molecule through the nitrogen atom and any other ring atom in said nitrogen containing heterocycle. The nitrogen containing heterocycle is optionally benzo-fused, and is preferably attached to the rest of the molecule through the nitrogen atom in said nitrogen containing heterocycle and any carbon atom in the fused benzene ring.

The term "substituted" means that one or more (e.g., one, two, three, or four) hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

If a substituent is described as being "optionally substituted," the substituent may be either (1) not substituted, or (2) substituted. If a carbon of a substituent is described as being optionally substituted with one or more of a list of substituents, one or more of the hydrogens on the carbon (to the extent there are any) may separately and/or together be replaced with an independently selected optional substituent. If a nitrogen of a substituent is described as being optionally substituted with one or more of a list of substituents, one or more of the hydrogens on the nitrogen (to the extent there are any) may each be replaced with an independently selected optional substituent.

If substituents are described as being "independently selected" from a group, each substituent is selected independent of the other(s). Each substituent therefore may be identical to or different from the other substituent(s).

As used herein, the term "one or more" means one or more than one (e.g., 2, 3, 4, 5 or 10) as reasonable.

As used herein, unless specified, the point of attachment of a substituent can be from any suitable position of the substituent.

When a bond to a substituent is shown to cross a bond connecting two atoms in a ring, then such substituent may be bonded to any of the ring-forming atoms in that ring that are substitutable.

The present invention also includes all pharmaceutically acceptable isotopically labeled compounds, which are identical to those of the present invention except that one or more atoms are replaced by an atom having the same atomic number, but an atomic mass or mass number different from the atomic mass or mass number which predominates in nature. Examples of isotopes suitable for inclusion in the compound of the present invention include, but are not limited to, isotopes of hydrogen, such as ²H, ³H; carbon, such as ¹¹C, ¹³C, and ¹⁴C; chlorine, such as ³⁶Cl; fluorine, such as ¹⁸F; iodine, such as ¹²³I and ¹²⁵I; nitrogen, such as ¹³N and ¹⁵N; oxygen, such as ¹⁵O, ¹⁷O, and ¹⁸O; phosphorus, such as ³²P; and sulfur, such as ³⁵S. Certain isotopically labeled compounds of the present invention, for example those incorporating a radioactive isotope, are useful in drug and/or substrate tissue distribution studies (e.g., assays). The radioactive isotopes tritium, i.e., ³H, and carbon-14, i.e., ¹⁴C, are particularly useful for this purpose in view of their ease of incorporation and ready means of detection. Substitution with positron-emitting isotopes, such as ¹¹C, ¹⁸F, ¹⁵O and ¹³N, can be useful in positron emission tomography (PET) studies for examining substrate receptor occupancy. Isotopically labeled compounds of the present invention can generally be prepared by processes analogous to those described in the accompanying Schemes and/or in the Examples and Preparations, by using an appropriate isotopically labeled reagent in place of the non-labeled reagent previously employed. Pharmaceutically acceptable solvates in accordance with the invention include those wherein the solvent of crystallization may be isotopically substituted, e.g., D₂O, acetone-*d₆*, or DMSO-*d₆*.

The term "stereoisomer "refers to isomers with at least one asymmetric center. A compound having one or more (e.g., one, two, three or four) asymmetric centers can give rise to a racemic mixture, single enantiomer, diastereomer mixture and individual diastereomer. Certain individual molecules may exist as geometric isomers (cis/trans). Similarly, the compound of the present invention may exist as a mixture of two or more structurally different forms in rapid equilibrium (generally referred to as tautomer). Typical examples of a tautomer include a keto-enol tautomer, phenol-keto tautomer, nitroso-oxime tautomer, imine-enamine tautomer and the like. It is to be understood that all such isomers and mixtures thereof in any proportion (such as 60%, 65%, 70%, 75%, 80%, 85%, 90%, 95%, 96%, 97%, 98%, and 99%) are encompassed within the scope of the present invention.

The chemical bonds of the compound of the present invention may be depicted herein using a solid line ( 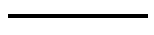 ), a solid wedge ( 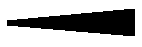 ), or a dotted wedge ( 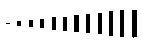 ). The use of a solid line to depict bonds to asymmetric carbon atoms is meant to indicate that all possible stereoisomers (e.g., specific enantiomers, racemic mixtures, *etc.*) at that carbon atom are included. The use of either a solid or dotted wedge to depict bonds to asymmetric carbon atoms is meant to indicate that the stereoisomer shown is present. When present in racemic compounds, solid and dotted wedges are used to define relative stereochemistry, rather than absolute stereochemistry. Unless stated otherwise, it is intended that the compound of the present invention can exist as stereoisomers, which include cis and trans isomers, optical isomers such as R and S enantiomers, diastereomers, geometric isomers, rotational isomers, conformational isomers, atropisomers, and mixtures thereof. The compound of the present invention may exhibit more than one type of isomerism, and consist of mixtures thereof (such as racemates and diastereomeric pairs).

The present invention includes all possible crystalline forms or polymorphs of the compound of the present invention, either as a single polymorph, or as a mixture of more than one polymorphs, in any ratio.

It also should be understood that, certain compounds of the present invention can be used for the treatment in a free form, or where appropriate, in a form of a pharmaceutically acceptable derivative. In the present invention, the pharmaceutically acceptable derivative includes, but is not limited to a pharmaceutically acceptable salt, ester, solvate, N-oxide, metabolite or prodrug, which can directly or indirectly provide the compound of the present invention or a metabolite or residue thereof after being administered to a patient in need thereof. Therefore, "the compound of the present invention" mentioned herein also means to encompass various derivative forms of the compound as mentioned above.

A pharmaceutically acceptable salt of the compound of the present invention includes an acid addition salt and a base addition salt thereof.

A suitable acid addition salt is formed from an acid which forms a pharmaceutically acceptable salt. Specific examples include acetate, adipate, aspartate, benzoate, besylate, bicarbonate/carbonate, bisulfate/sulfate, borate, camphorsulfonate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

A suitable base addition salt is formed from a base which forms a pharmaceutically acceptable salt. Specific examples include aluminum, arginine, benzathine, calcium, choline, diethylamine, diolamine, glycine, lysine, magnesium, meglumine, olamine, potassium, sodium, tromethamine and zinc salts.

For a review on suitable salts, see "Handbook of Pharmaceutical Salts: Properties, Selection, and Use" by Stahl and Wermuth (Wiley-VCH, 2002). The method for preparing a pharmaceutically acceptable salt of the compound of the present invention is known to a person skilled in the art.

As used herein, the term "ester" refers to those derived from the compounds of the various formulae in the present application, which include physiologically-hydrolyzable esters (which may be hydrolyzed under physiological conditions to release the compounds of the present invention in the form of free acids or alcohols). The compound of the present invention itself may be an ester as well.

The compound of the present invention can exist as a solvate (preferably a hydrate), wherein the compound of the present invention contains a polar solvent, in particular water, methanol or ethanol for example, as a structural element of the crystal lattice of the compound. The amount of the polar solvent, in particular water, may exist in a stoichiometric or non-stoichiometric ratio.

As can be appreciated by a person skilled in the art, not all nitrogen containing heterocycles can form N-oxides since the nitrogen requires an available lone-pair electron for oxidation to the oxide; a person skilled in the art will recognize those nitrogen containing heterocycles which can form N-oxides. A person skilled in the art will also recognize that tertiary amines can form N-oxides. Synthetic methods for the preparation of N-oxides of heterocycles and tertiary amines are well known to a person skilled in the art, and they include the oxidation of heterocycles and tertiary amines with peroxy acids such as peracetic acid and m-chloroperbenzoic acid (MCPBA), hydrogen peroxide, alkyl hydroperoxides such as *tert*-butyl hydroperoxide, sodium perborate, and dioxiranes such as dimethyldioxirane. These methods for the preparation of N-oxides have been extensively described and reviewed in literatures, see e.g., T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

The metabolite of the compound of the present invention, namely a substance formed *in vivo* upon administration of the compound of the present invention, is also included within the scope of the present invention. Such a product may result e.g., from the oxidation, reduction, hydrolysis, amidation, de-amidation, esterification, enzymolysis, and the like, of the administered compound. Accordingly, the present invention encompasses the metabolite of the compound of the present invention, including a compound produced by a method comprising contacting the compound of the present invention with a mammal for a period of time sufficient to result in a metabolic product thereof.

Also within the scope of the present invention is a prodrug of the compound of the invention, which is certain derivative of the compound of the invention that may have little or no pharmacological activity itself, but can, when administered into or onto the body, be converted into the compound of the invention having the desired activity, for example, by hydrolytic cleavage. In general, such prodrug will be a functional derivative of the compound which is readily converted *in vivo* into the compound with desired therapeutic activity. Further information on the use of the prodrug may be found in "Pro-drugs as Novel Delivery Systems", Vol. 14, ACS Symposium Series (T. Higuchi and V. Stella). The prodrug in accordance with the invention can, for example, be produced by replacing appropriate functionalities present in the compound of the present invention with certain moieties known to those skilled in the art as "pro-moieties" as described, for example, in "Design of Prodrugs" by H. Bundgaard (Elsevier, 1985).

The present invention further encompasses the compound of the present invention having a protecting group. During any of the processes for preparation of the compound of the present invention, it may be necessary and/or desirable to protect sensitive or reactive groups on any of the molecules concerned, thereby resulting in the chemically protected form of the compound of the present invention. This may be achieved by means of conventional protecting groups, e.g., those described in T.W. Greene & P.G.M. Wuts, Protective Groups in Organic Synthesis, John Wiley & Sons, 1991, which is incorporated herein by reference. The protecting groups may be removed at a convenient subsequent stage using methods known from the art.

The term "about" refers to a range within ±10%, preferably within ±5%, and more preferably within ±2% of the specified value.

The term "effective amount" refers to an amount sufficient to achieve the desired therapeutic effect, under the conditions of administration, and it causes an improvement in the pathological symptoms, disease progression, physiological conditions associated with or induces resistance to succumbing to the afore mentioned disorders.

As used herein, the term "pneumoconiosis" refers to a systemic disease caused by long-term inhalation of industrial dust (particulate matter) during occupational activities and its retention in the lungs. Based on the type of inhaled dust, pneumoconiosis may be classified into inorganic pneumoconiosis and organic pneumoconiosis. Pneumoconiosis caused by inhalation of inorganic dust is referred to as inorganic pneumoconiosis, which constitutes the majority of pneumoconiosis cases. Pneumoconiosis resulting from the inhalation of organic dust is known as organic pneumoconiosis, such as byssinosis or farmer's lung, *etc.*

Unless otherwise specified, the term "treating" as used herein means reversing, alleviating, inhibiting, delaying, or blocking the progression of a disorder or condition to which such term applies, or one or more symptoms thereof.

As used herein, the term "subject" includes a human or non-human animal. An exemplary human subject includes a human subject having a disease (such as one described herein) (referred to as a patient), or a normal subject. The term "non-human animal" as used herein includes all vertebrates, such as non-mammals (e.g., birds, amphibians, reptiles) and mammals, such as non-human primates, livestock and/or domesticated animals (such as sheep, dog, cat, cow, pig and the like).

In some embodiments, the present disclosure provides a method for the prophylaxis, alleviation, mitigation, and/or treatment of pneumoconiosis, comprising administering to a subject in need thereof an effective amount of a compound of Formula (I) or a pharmaceutically acceptable salt, ester, stereoisomer, polymorph, solvate, N-oxide, isotopically labeled compound, metabolite or prodrug thereof: 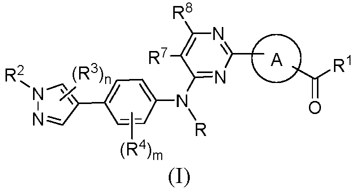 wherein:
ring A is 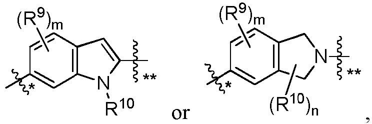 the above group is attached to the pyrimidine ring at either of the two positions labeled * or **, and is attached to the carbonyl group at the other position;
R is selected from the group consisting of H and C₁₋₆ alkyl;
R¹ is 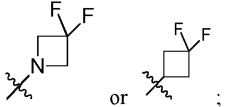
R² is selected from the group consisting of H and C₁₋₆ alkyl;
R³, R⁴, R⁷ and R⁸, at each occurrence, are each independently selected from the group consisting of H, halogen, -NR⁵R⁶, -OH, C₁₋₆ alkyl and -OR⁵;
R⁹ and R¹⁰, at each occurrence, are each independently selected from the group consisting of H, halogen, C₁₋₆ alkyl, C₂₋₆ alkenyl, C₃₋₁₀ cyclic hydrocarbyl, 3-10-membered heterocyclyl, C₆₋₁₀ aryl, 5-14-membered heteroaryl, C₆₋₁₂ aralkyl, -C(=O)R⁵ and -C₁₋₆ alkylene-O(P=O)(OH)₂;
the above alkylene, alkyl, alkenyl, cyclic hydrocarbyl, heterocyclyl, aryl, heteroaryl and aralkyl, at each occurrence, are each optionally substituted with one or more substituents independently selected from the group consisting of halogen, C₁₋₆ alkyl and -OR⁵;
R⁵ and R⁶, at each occurrence, are each independently selected from the group consisting of H, C₁₋₆ alkyl, C₃₋₁₀ cyclic hydrocarbyl, 3-10-membered heterocyclyl, C₆₋₁₀ aryl, 5-14-membered heteroaryl and C₆₋₁₂ aralkyl;
m, at each occurrence, is each independently an integer of 0, 1, 2 or 3; and
n, at each occurrence, is each independently an integer of 0, 1 or 2.

In preferred embodiments, ring A is 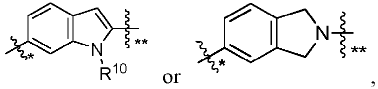 the above group is attached to the pyrimidine ring at the position labeled *, and is attached to the carbonyl group at the position labeled **, wherein R¹⁰ is selected from the group consisting of H and C₁₋₆ alkyl, preferably is H or methyl.

In preferred embodiments, ring A preferably is 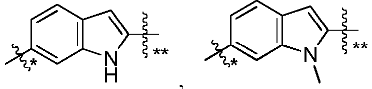 or 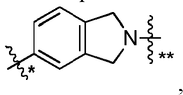 the above group is attached to the pyrimidine ring at the position labeled *, and is attached to the carbonyl group at the position labeled **.

In preferred embodiments, R is H.

In preferred embodiments, R² is H.

In preferred embodiments, R⁵ and R⁶, at each occurrence, are each independently selected from the group consisting of H, methyl and ethyl.

In preferred embodiments, R³, R⁴, R⁷ and R⁸, at each occurrence, are each independently selected from the group consisting of H, F, Cl, Br, I, -NH₂, -OH, methyl, trifluoromethyl, -CH₂-Ph, methoxy, ethoxy and -CH₂OCH₃.

In preferred embodiments, R³ is H.

In preferred embodiments, R⁴ is selected from the group consisting of H and halogen (e.g., F, Cl, Br or I), preferably is H or F.

In preferred embodiments, R⁷ is selected from the group consisting of H and halogen (e.g., F, Cl, Br or I), preferably is H or F.

In preferred embodiments, R⁸ is H.

In preferred embodiments, R⁹ and R¹⁰, at each occurrence, are each independently selected from the group consisting of H, F, Cl, Br, methyl, ethyl, n-propyl, isopropyl, vinyl, cyclopropyl, cyclobutyl, cyclopentyl, oxetanyl, monofluoromethyl, difluoromethyl, trifluoromethyl, acetyl, -CH₂CHF₂, -CH₂OH, -CH₂OCH₃, -CH₂CH₂OCH₃, -CH₂-O(P=O)(OH)₂,

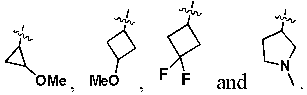

In preferred embodiments, R⁹, at each occurrence, is each independently selected from the group consisting of H, C₁₋₆ alkyl, C₃₋₁₀ cyclic hydrocarbyl, 3-10-membered heterocyclyl, C₆₋₁₀ aryl, 5-14-membered heteroaryl and C₆₋₁₂ aralkyl, preferably is H.

In preferred embodiments, R¹⁰, at each occurrence, is each independently selected from the group consisting of H and C₁₋₆ alkyl, preferably is H, methyl, ethyl, n-propyl or isopropyl, and most preferably is H or methyl.

In preferred embodiments, the present disclosure provides a method for the prophylaxis, alleviation, mitigation, and/or treatment of pneumoconiosis, comprising administering to a subject in need thereof an effective amount of a compound of Formula (II) or a pharmaceutically acceptable salt, ester, stereoisomer, polymorph, solvate, N-oxide, isotopically labeled compound, metabolite or prodrug thereof: 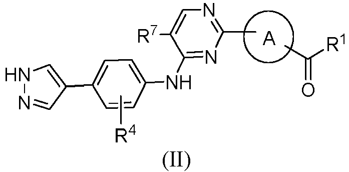 wherein each of the groups is as defined above.

In preferred embodiments, the present disclosure provides a method for the prophylaxis, alleviation, mitigation, and/or treatment of pneumoconiosis, comprising administering to a subject in need thereof an effective amount of a compound of Formula (III) or a pharmaceutically acceptable salt, ester, stereoisomer, polymorph, solvate, N-oxide, isotopically labeled compound, metabolite or prodrug thereof: 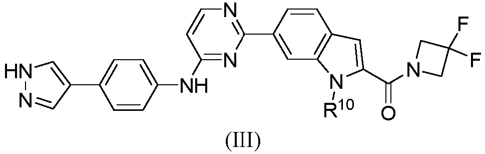 wherein R¹⁰ is H or methyl, preferably is methyl.

In preferred embodiments, the compound has the following structure:

| **Compound No.** | **Structure** |
|---|---|
| **006** | 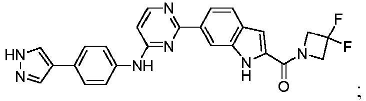 |
| **007** | 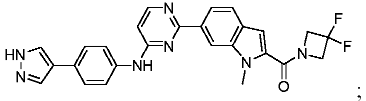 |
| **008** | 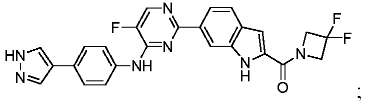 |
| **009** | 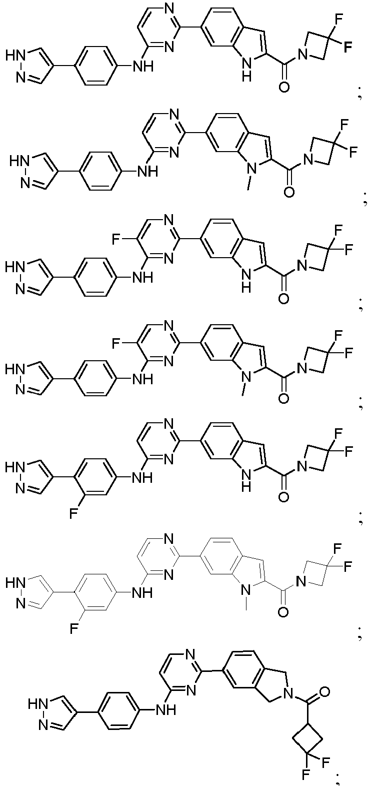 |
| **010** | 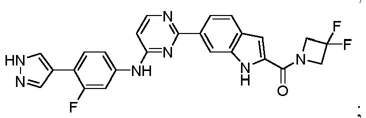 |
| **011** | 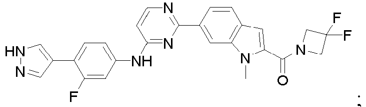 |
| **020** | 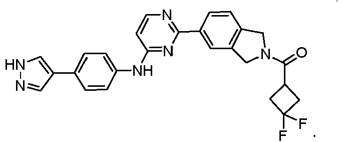 |
| **021** | 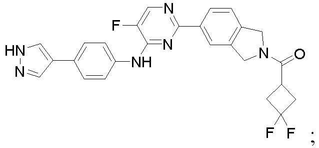 or |
| **022** | 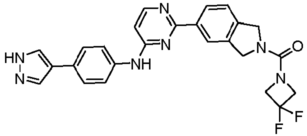 |

In some embodiments, the compounds are prepared according to the methods disclosed in WO 2019/001572 A1 (incorporated herein by reference).

In some embodiments, the compound of Formula (I), (II) or (III) or a pharmaceutically acceptable salt, ester, stereoisomer, polymorph, solvate, N-oxide, isotopically labeled compound, metabolite or prodrug thereof is administered in an amount of about 0.005 mg/day to about 5000 mg/day, e.g., in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500 or 5000 mg/day.

In some embodiments, the compound of Formula (I), (II) or (III) or a pharmaceutically acceptable salt, ester, stereoisomer, polymorph, solvate, N-oxide, isotopically labeled compound, metabolite or prodrug thereof is administered in an amount of about 1 ng/kg to about 200 mg/kg, about 1 µg/kg to about 100 mg/kg or about 1 mg/kg to about 50 mg/kg body weight per day, e.g., is administered in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg or about 300 mg/kg body weight per day.

In some embodiments, the daily dose of the compound of Formula (I), (II) or (III) or a pharmaceutically acceptable salt, ester, stereoisomer, polymorph, solvate, N-oxide, isotopically labeled compound, metabolite or prodrug thereof is administered at one time or is administered in two, three or four doses.

In some embodiments, the compound of Formula (I), (II) or (III) or a pharmaceutically acceptable salt, ester, stereoisomer, polymorph, solvate, N-oxide, isotopically labeled compound, metabolite or prodrug thereof is administered continuously for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 26 days, at least 27 days, at least 28 days, at least 29 days, at least 1 month, at least 2 months, at least 3 months, at least 4 months, at least 5 months, at least 6 months, at least 1 year, or at least 2 years.

In some embodiments, the compound of Formula (I), (II) or (III) or a pharmaceutically acceptable salt, ester, stereoisomer, polymorph, solvate, N-oxide, isotopically labeled compound, metabolite or prodrug thereof is administered for one or more (e.g., 1, 2, 3, 4, 5, 6, 7, 8, 9 or 10) courses of treatment, wherein each course of treatment lasts for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 26 days, at least 27 days, at least 28 days, at least 29 days, at least 30 days, at least 35 days, at least 40 days, at least 45 days or at least 50 days; and the interval between every two courses of treatment is 0, 1, 2, 3, 4, 5, 6, 7, 8, 9, 10 days, two weeks, three weeks, or four weeks.

In some embodiments, the compound of Formula (I), (II) or (III) or a pharmaceutically acceptable salt, ester, stereoisomer, polymorph, solvate, N-oxide, isotopically labeled compound, metabolite or prodrug thereof is administered through injection (e.g., intravenous, intraarterial, subcutaneous, intraperitoneal, intramuscular injection, including dripping), or transdermal administration, or is administered via oral, buccal, nasal, transmucosal, topical, as an ophthalmic formulation, or via inhalation.

In some embodiments, the compound of Formula (I), (II) or (III) or a pharmaceutically acceptable salt, ester, stereoisomer, polymorph, solvate, N-oxide, isotopically labeled compound, metabolite or prodrug thereof is administered in a dosage form selected from the group consisting of tablet, capsule, lozenge, hard candy, powder, spray, cream, salve, suppository, gel, paste, lotion, ointment, aqueous suspensions, injectable solution, elixir, and syrup.

In some embodiments, the present disclosure provides use of the compound of Formula (I), (II) or (III) or a pharmaceutically acceptable salt, ester, stereoisomer, polymorph, solvate, N-oxide, isotopically labeled compound, metabolite or prodrug thereof in the manufacture of a medicament for the prophylaxis, alleviation, mitigation, and/or treatment of pneumoconiosis.

In some embodiments, the present disclosure provides the compound of Formula (I), (II) or (III) or a pharmaceutically acceptable salt, ester, stereoisomer, polymorph, solvate, N-oxide, isotopically labeled compound, metabolite or prodrug thereof for use in the prophylaxis, alleviation, mitigation, and/or treatment of pneumoconiosis.

In some embodiments, the pneumoconiosis is inorganic pneumoconiosis or organic pneumoconiosis.

In preferred embodiments, the pneumoconiosis is selected from the group consisting of silicosis, coal workers' pneumoconiosis, graphite pneumoconiosis, carbon black pneumoconiosis, asbestosis, talc pneumoconiosis, cement pneumoconiosis, mica pneumoconiosis, potter's pneumoconiosis, aluminosis, welder's pneumoconiosis, and foundry worker's pneumoconiosis.

In some embodiments, the prophylaxis, alleviation, mitigation, and/or treatment of pneumoconiosis comprises the prophylaxis, alleviation, mitigation, and/or treatment of the following symptoms: cough, expectoration, chest pain, and/or dyspnea.

The present disclosure encompasses any combination of the above embodiments.

### Example

In order to make the objects and technical solutions of the invention clearer, the invention will be further described below with reference to specific examples. It should be understood that the following examples are only intended for illustrating the invention and are not to be understood as limiting the scope of the invention. Further, specific experimental methods not mentioned in the following examples are carried out in accordance with conventional experimental methods.

### Example 1

After acclimatization, 6-8-week-old male C57BL/6J mice were intratracheally instilled 40 µL of SiO₂ suspension (600 mg/kg) on Day 1 (D1) to establish the silicosis model. On the same day (D1), the animals were randomly divided into 3 groups based on body weight and administered either the vehicle (model group) or Compound 007 (100 mg/kg or 150 mg/kg, once daily) via oral gavage for 28 consecutive days. A normal control group was also established, intratracheally instilled 40 µL of saline on D1 and orally administered sterile water. No mortality was observed in any group during the dosing period.

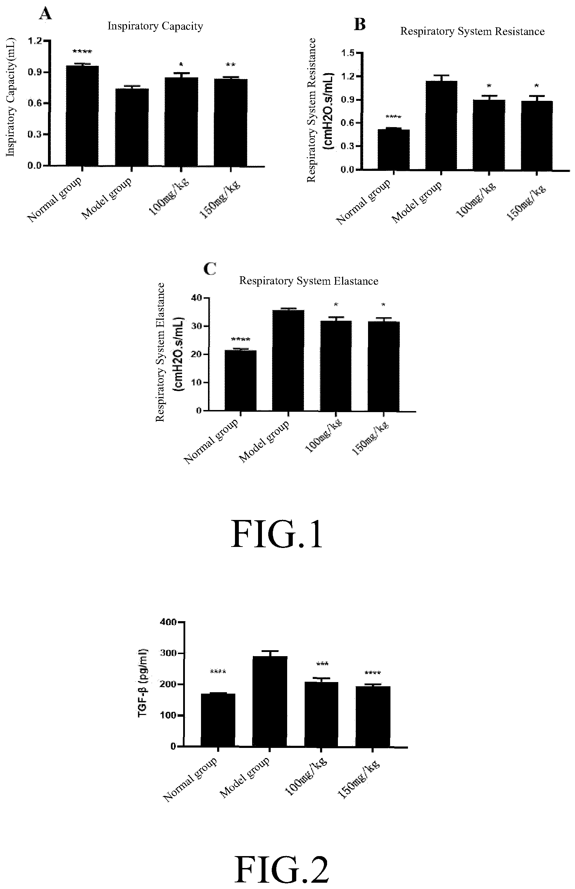

Two hours after the final administration, mouse pulmonary function indices were measured. As shown in Figure 1, compared with the model group, daily administration of Compound 007 at 100 mg/kg and 150 mg/kg significantly increased inspiratory capacity (p < 0.05, p < 0.01), reduced respiratory system resistance (p < 0.05, p < 0.05), and decreased respiratory system elastance (p < 0.05, p < 0.05).

After the administration was completed, the animals were euthanized, and bronchoalveolar lavage fluid (BALF) was collected from randomly selected animals in each group for TGF-β detection. The results are shown in Figure 2, which demonstrate that daily administration of Compound 007 at 100 mg/kg and 150 mg/kg significantly reduced TGF-β levels (Figure 2, p < 0.001, p < 0.0001).

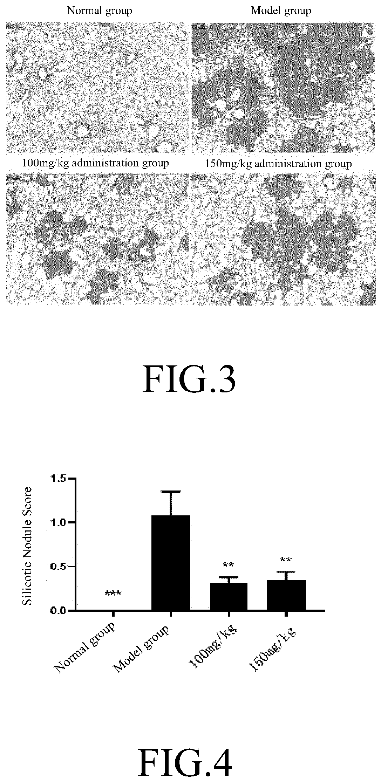

Lung tissues from all mice were collected, sectioned, and subjected to Masson's Trichrome staining and H&E staining for silicotic nodule scoring and inflammation scoring. Silicotic nodule scoring was performed using the King's five-point scale [Zhujie Cao, et al., Acta Pharmacologica Sinica, 2021], where the severity of silicotic nodules in lung tissue was scored from 0 to 5, with higher scores indicating more severe nodulation. H&E pathological scoring followed Szapiel's inflammation scoring criteria, where the degree of inflammatory cell infiltration in lung tissue was scored from 0 to 3, with higher scores indicating greater inflammation in the lung tissue (0: no inflammation or alveolitis; 1: mild, mononuclear cell infiltration with thickened alveolar septa, localized involvement, pleural lesions involving less than 20% of the lung, and well-preserved alveolar structure; 2: moderate, more extensive alveolitis involving 20%-50% of the lung, primarily pleural lesions; 3: severe, diffuse alveolitis involving over 50% of the lung, occasionally with mononuclear cell infiltration in the alveoli and interstitium and/or some hemorrhagic areas within the alveoli). Results are shown in Tables 1-2 and Figures 3-6.

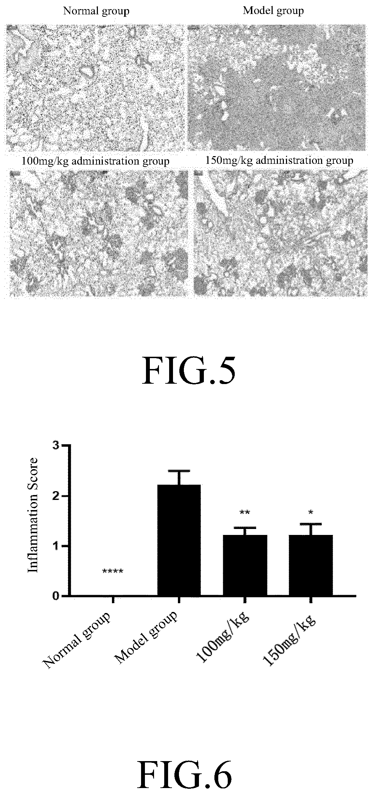

The results demonstrate that daily administration of Compound 007 at 100 mg/kg and 150 mg/kg reduced silica-induced nodule formation and collagen fiber deposition, significantly lowering silicotic nodule scores (Figures 3-4, p < 0.01, p < 0.01), as shown in Table 1. Additionally, Compound 007 at 100 mg/kg and 150 mg/kg markedly improved lung tissue inflammation in mice and reduced inflammation scores (p < 0.01 and p < 0.05) (Figures 5-6, Table 2).

**Table 1 Silica Nodule Scores in Lung of Silicosis Model Mice in Each Group**

| **Group** | **Silicotic Nodule Score (Mean ± SEM)** | **p-value (vs. Model Group)** |
|---|---|---|
| Normal Group | 0 | <0.001 |
| Model Group | 1.08±0.27 | / |
| Compound 007 100 mg/kg administration group | 0.32±0.06 | <0.01 |
| Compound 007 150 mg/kg administration group | 0.35±0.09 | <0.01 |

**Table 2 Inflammation Scores in Lung Tissues of Silicosis Model Mice in Each Group**

| **Group** | **Inflammation Score (Mean ± SEM)** | **p-value (vs. Model Group)** |
|---|---|---|
| Normal Group | 0 | <0.0001 |
| Model Group | 2.22±0.28 | / |
| Compound 007 100 mg/kg administration group | 1.22±0.15 | <0.01 |
| Compound 007 150 mg/kg administration group | 1.22±0.22 | <0.05 |

In summary, in the silica-induced pulmonary nodule mouse model, Compound 007 improved mouse pulmonary function, specifically by increasing inspiratory capacity and reducing respiratory system resistance and respiratory system elastance. Furthermore, Compound 007 reduced silicotic nodule scores, inflammation scores in mouse lungs and lowered TGF-β levels in BALF.

Various modifications to the invention in addition to those described herein will become apparent to those skilled in the art from the foregoing description. Such modifications are intended to fall within the scope of the appended claims. Each reference, including all patents, applications, journal articles, books and any other disclosure, referred to herein is hereby incorporated by reference in its entirety.

## Claims

1. A method for the prophylaxis, alleviation, mitigation, and/or treatment of pneumoconiosis, comprising administering to a subject in need thereof an effective amount of a compound of Formula (I) or a pharmaceutically acceptable salt, ester, stereoisomer, polymorph, solvate, N-oxide, isotopically labeled compound, metabolite or prodrug thereof:
wherein: 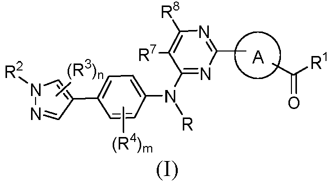
ring A is 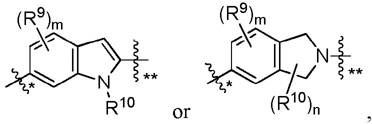 the above group is attached to the pyrimidine ring at either of the two positions labeled * or **, and is attached to the carbonyl group at the other position;
preferably, ring A is 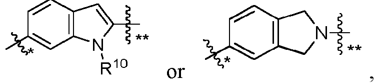 the above group is attached to the pyrimidine ring at the position labeled *, and is attached to the carbonyl group at the position labeled **,
R is selected from the group consisting of H and C₁₋₆ alkyl; 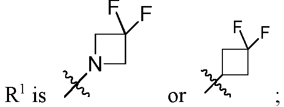
R² is selected from the group consisting of H and C₁₋₆ alkyl;
R³, R⁴, R⁷ and R⁸, at each occurrence, are each independently selected from the group consisting of H, halogen (e.g., F, Cl, Br or I), -NR⁵R⁶, -OH, C₁₋₆ alkyl and -OR⁵;
R⁹ and R¹⁰, at each occurrence, are each independently selected from the group consisting of H, halogen, C₁₋₆ alkyl (e.g., methyl), C₂₋₆ alkenyl, C₃₋₁₀ cyclic hydrocarbyl, 3-10-membered heterocyclyl, C₆₋₁₀ aryl, 5-14-membered heteroaryl, C₆₋₁₂ aralkyl, -C(=O)R⁵ and -C₁₋₆ alkylene-O(P=O)(OH)₂;
the above alkylene, alkyl, alkenyl, cyclic hydrocarbyl, heterocyclyl, aryl, heteroaryl and aralkyl, at each occurrence, are each optionally substituted with one or more substituents independently selected from the group consisting of halogen, C₁₋₆ alkyl and -OR⁵;
R⁵ and R⁶, at each occurrence, are each independently selected from the group consisting of H, C₁₋₆ alkyl, C₃₋₁₀ cyclic hydrocarbyl, 3-10-membered heterocyclyl, C₆₋₁₀ aryl, 5-14-membered heteroaryl and C₆₋₁₂ aralkyl;
m, at each occurrence, is each independently an integer of 0, 1, 2 or 3; and
n, at each occurrence, is each independently an integer of 0, 1 or 2.

2. The method according to claim 1, wherein the compound has the structure of Formula (II): 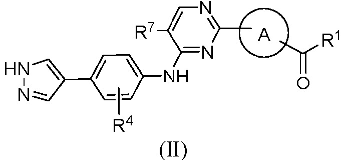
wherein each of the groups is as defined in claim 1;
preferably, the compound has the structure of Formula (III): 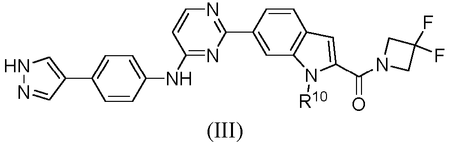
wherein R¹⁰ is H or C₁₋₆ alkyl, preferably is H or methyl, and more preferably is methyl.

3. The method according to claim 1 or 2, wherein the compound has the following structure:
| **Compound No.** | **Structure** |
|---|---|
| **006** | 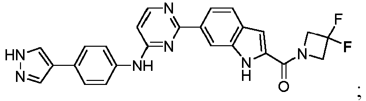 |
| **007** | 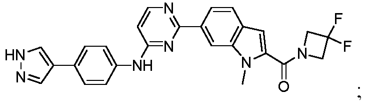 |
| **008** | 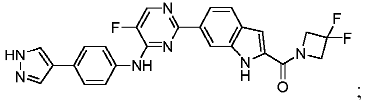 |
| **009** | 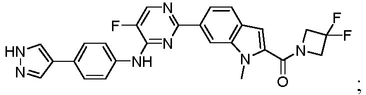 |
| **010** | 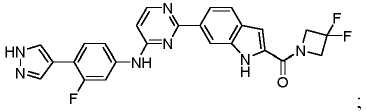 |
| **011** | 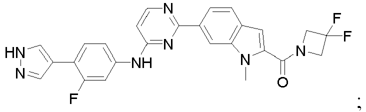 |
| **020** | 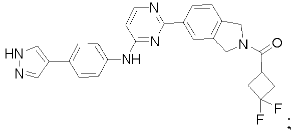 |
| **021** | 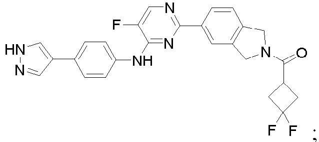 or |
| **022** | 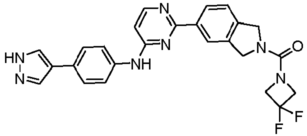 |

4. The method according to any one of claims 1 to 3, wherein the compound of Formula (I) or a pharmaceutically acceptable salt, ester, stereoisomer, polymorph, solvate, N-oxide, isotopically labeled compound, metabolite or prodrug thereof is administered in an amount of about 0.005 mg/day to about 5000 mg/day, e.g., in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500 or 5000 mg/day.

5. The method according to any one of claims 1 to 3, wherein the compound of Formula (I) or a pharmaceutically acceptable salt, ester, stereoisomer, polymorph, solvate, N-oxide, isotopically labeled compound, metabolite or prodrug thereof is administered in an amount of about 1 ng/kg to about 200 mg/kg, about 1 µg/kg to about 100 mg/kg or about 1 mg/kg to about 50 mg/kg body weight per day, e.g., is administered in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg or about 300 mg/kg body weight per day.

6. The method according to any one of claims 1 to 5, wherein the daily dose of the compound of Formula (I) or a pharmaceutically acceptable salt, ester, stereoisomer, polymorph, solvate, N-oxide, isotopically labeled compound, metabolite or prodrug thereof is administered at one time or is administered in two, three or four doses.

7. The method according to any one of claims 1 to 6, wherein the compound of Formula (I) or a pharmaceutically acceptable salt, ester, stereoisomer, polymorph, solvate, N-oxide, isotopically labeled compound, metabolite or prodrug thereof is administered continuously for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 26 days, at least 27 days, at least 28 days, at least 29 days, at least 1 month, at least 2 months, at least 3 months, at least 4 months, at least 5 months, at least 6 months, at least 1 year, or at least 2 years.

8. The method according to any one of claims 1 to 7, wherein the compound of Formula (I) or a pharmaceutically acceptable salt, ester, stereoisomer, polymorph, solvate, N-oxide, isotopically labeled compound, metabolite or prodrug thereof is administered for one or more (e.g., 1, 2, 3, 4, 5, 6, 7, 8, 9 or 10) courses of treatment, wherein each course of treatment lasts for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 26 days, at least 27 days, at least 28 days, at least 29 days, at least 30 days, at least 35 days, at least 40 days, at least 45 days or at least 50 days; and the interval between every two courses of treatment is 0, 1, 2, 3, 4, 5, 6, 7, 8, 9, 10 days, two weeks, three weeks, or four weeks.

9. The method according to any one of claims 1 to 8, wherein the compound of Formula (I) or a pharmaceutically acceptable salt, ester, stereoisomer, polymorph, solvate, N-oxide, isotopically labeled compound, metabolite or prodrug thereof is administered through injection (e.g., intravenous, intraarterial, subcutaneous, intraperitoneal, intramuscular injection, including dripping), or transdermal administration, or is administered via oral, buccal, nasal, transmucosal, topical, as an ophthalmic formulation, or via inhalation.

10. The method according to any one of claims 1 to 9, wherein the compound of Formula (I) or a pharmaceutically acceptable salt, ester, stereoisomer, polymorph, solvate, N-oxide, isotopically labeled compound, metabolite or prodrug thereof is administered in a dosage form selected from the group consisting of tablet, capsule, lozenge, hard candy, powder, spray, cream, salve, suppository, gel, paste, lotion, ointment, aqueous suspensions, injectable solution, elixir, and syrup.

11. The method according to any one of claims 1-10, wherein the pneumoconiosis is inorganic pneumoconiosis or organic pneumoconiosis;
preferably, the pneumoconiosis is selected from the group consisting of silicosis, coal workers' pneumoconiosis, graphite pneumoconiosis, carbon black pneumoconiosis, asbestosis, talc pneumoconiosis, cement pneumoconiosis, mica pneumoconiosis, potter's pneumoconiosis, aluminosis, welder's pneumoconiosis, and foundry worker's pneumoconiosis.

12. The method according to any one of claims 1-11, wherein the prophylaxis, alleviation, mitigation, and/or treatment of pneumoconiosis comprises the prophylaxis, alleviation, mitigation, and/or treatment of the following symptoms: cough, expectoration, chest pain, and/or dyspnea.
